# EUROPEAN PATENT APPLICATION

(11) **EP 3 936 865 A1**
(43) Date of publication of application: **12.01.2022**
(21) Application number: 20784400.2
(22) Date of filing: 26.02.2020
(51) Int. Cl.: G01N 33/53

(54) **DEPRESSION EVALUATION METHOD AND METHOD FOR EVALUATING DEPRESSION TREATMENT METHOD, AND DATA ACQUISITION METHOD FOR EVALUATING DEPRESSION OR RESULT OF DEPRESSION TREATMENT METHOD**

(30) Priority: 29.03.2019 JP 2019067051
(71) Applicant: Japan Tobacco Inc., Tokyo 105-6927 (JP); Shinshu University, Matsumoto-shi, Nagano 390-8621 (JP)
(72) Inventor: YAMAGUCHI, Masaki, Ueda-shi, Nagano 386-8567 (JP); WASHIDUKA Shinsuke, Matsumoto-shi, Nagano 390-8621 (JP); SASAYAMA, Daimei, Matsumoto-shi, Nagano 390-8621 (JP); SEKINE, Takayasu, Tokyo 105-6927 (JP); TSUKUI, Shu, Tokyo 105-6927 (JP)
(74) Representative: Isarpatent
(86) International application number: PCT/JP2020/007777
(87) International publication number: WO 2020/202923

(57) **Abstract**

A method for evaluating whether an evaluation subject suffers from depression or whether an evaluation subject has possibility of development of depression, the method including evaluating whether the evaluation subject suffers from depression or whether the evaluation subject has possibility of development of depression by using as an indicator an amount of a cytokine in a sample derived from the evaluation subject.

## Description

### Technical Field

The present invention relates to a method for evaluating whether an evaluation subject suffers from depression or whether an evaluation subject has possibility of development of depression, a method for evaluating an effect of a therapy for depression in an evaluation subject, and a method for acquiring data for evaluating whether an evaluation subject suffers from depression or whether an evaluation subject has possibility of development of depression, or data for evaluating an effect of a therapy for depression in an evaluation subject.

### Background Art

Depression is one kind of mood disorders. It is a disorder causing, for example, feelings of helplessness and anxiety, characterized by decreased attention, depressive symptoms, loss of vitality, loss of interest, change in appetite (mainly decreases but may increase), change in sleep state, persistent feelings of, for example, sadness and anxiety, and symptoms such as declines in physical functions and mental functions.

Depression may be classified into exogenous (somatogenic) depression, endogenous depression, and psychogenic (character environmental) depression. Somatogenic depression refers to depression caused by physical diseases attributed to the brain and hormone secretion mechanism. Endogenous depression refers to so-called generally known depression. Psychogenic depression refers to where patients' characters and living environments are strongly related to their depressive states.

The depression (major depressive disorder) according to DSM-5 (the diagnostic criteria by the American psychiatric association) is classified based on symptoms by excluding depression due to physiological actions of substances and other medical diseases (so-called exogenous depression).

In the present invention, all of them are collectively referred to as "depression".

According to the numbers by diseases of patients who visit medical institutions issued by the Ministry of Health, Labour and Welfare, the number of the patients who visit there for depression is the largest compared with, for example, schizophrenia and dementia. Depression is considered as a social issue because the patients who visit there have been increasing year by year (according to the statistics of the WHO, it is expected that over 3 million patients with depression are in Japan).

The mechanism of depression is not still clear, and there is no curative therapy. In many cases, therapy proceeds based on combination of behavior therapy and drug therapy.

Examples of drugs used for drug therapy include SSRI (selective serotonin reuptake inhibitor), SNRI (serotonin and noradrenaline reuptake inhibitor), NaSSA (noradrenergic and specific serotonergic antidepressant), and tricyclic- and tetracyclic-antidepressants, which act on serotonin and noradrenaline.

However, some data say that a therapy using the above antidepressant alone improves the symptoms in about 50% of the patients, and ameliorates (removes the symptoms) in about 30% of the whole. Not a few patients feel that their symptoms do not become better even if they continue the therapy according to the instructions of their doctors. Therefore, "enhanced therapy" is promising, which uses an antidepressant and an atypical antipsychotic in combination.

Problems with drug therapy include side effects of antidepressants and difference in strength of the effect depending on patients. Examples of the side effects include, for SSRI-based drugs, neuropsychiatric side effects such as inattention, side effects in the gastrointestinal system such as diarrhea and vomiting, and side effects in the circulatory system such as blood pressure reduction and bradycardia. Some reports point out an increased risk of suicidal ideation and suicide attempts, and care should be taken for administration of the drugs.

Judgment of an effect of drug therapy requires to diagnose response and amelioration. In clinical sites, rating scales by evaluator's interviews such as the Hamilton depression rating scale and MADRS (subscale of comprehensive psychopathological rating scale) have been used, and in fact, no objective evaluation system has been established.

Diagnosis of depression is generally made by, for example, interviews similar to many psychiatric disorders. The above Hamilton depression rating scale and MADRS, and others rating scales are for aiding diagnosis. However, there is still no biological indicator, and therefore misdiagnosis often occurs and an objective indicator is demanded.

So far, various methods other than interviews have been proposed, including a diagnosis method using cardiac rate patterns (see, for example, PTL 1), a determination method using as an indicator the expression level of a specific gene (see, for example, PTL 2), and a determination method through *in vivo* imaging of the brain (see, for example, PTLs 3 and 4).

However, any of them have not yet been put into clinical use.

In addition, studies have been made for biomarkers of depression, and many biomarker candidates have been proposed, which include fibrinogen (see, for example, PTL 5), enteric bacteria (see, for example, PTL 6), γ-aminobutyric acid (see, for example, PTL 7), and factors related to latent infection of herpesvirus (see, for example, PTL 8).

However, any of them have not yet been put into clinical use.

Therefore, there is currently a strong demand for rapid provision of a new technique that can highly accurately evaluate whether a subject suffers from depression or whether a subject has possibility of development of depression, and whether a therapy for depression such as the behavior therapy and the drug therapy is effective to patients who have already suffered from depression.

### Citation List

### Patent Literature

PTL 1: Japanese Patent Application Laid-Open (JP-A) No. 2001-518823
PTL 2: JP-ANo. 2009-112266
PTL 3: JP-ANo. 2011-523638
PTL 4: JP-ANo.2011-523637
PTL 5: International Publication No. WO2009/096502
PTL 6: International Publication No. WO2016/167365
PTL 7: International Publication No. WO2017/082103
PTL 8: International Publication No. WO2009/041501

### Summary of Invention

### Technical Problem

The present invention aims to solve the various problems in the art and achieve the following object. Specifically, an object of the present invention is to provide: an evaluation method that can highly accurately evaluate whether a subject suffers from depression or whether a subject has possibility of development of depression; an evaluation method that can highly accurately evaluate whether the therapy for depression implemented to the patient who has already suffered from depression is effective; and a method for acquiring data for highly accurately evaluating whether a subject suffers from depression or whether a subject has possibility of development of depression, or whether the therapy for depression implemented to the patient who has already suffered from depression is effective.

### Solution to Problem

The present invention conducted intensive studies to achieve the above object. As a result, they have found that the amount of a specific cytokine in a sample derived from a patient with depression is significantly different from the amount of a corresponding cytokine in a sample derived from a healthy subject.

The present invention is based on the above finding obtained by the present inventors, and means for solving the above problems are as follows.
<1> A method for evaluating whether an evaluation subject suffers from depression or whether an evaluation subject has possibility of development of depression, the method including
   evaluating whether the evaluation subject suffers from depression or whether the evaluation subject has possibility of development of depression by using as an indicator an amount of a cytokine in a sample derived from the evaluation subject.
<2> A method for evaluating an effect of a therapy for depression in an evaluation subject, the method including
   evaluating the effect of the therapy for depression by using as an indicator an amount of a cytokine in a sample derived from an evaluation subject.
<3> A method for acquiring data for evaluating whether an evaluation subject suffers from depression or whether an evaluation subject has possibility of development of depression, or data for evaluating an effect of a therapy for depression in an evaluation subject, the method including
   measuring an amount of a cytokine in a sample derived from the evaluation subject.

### Advantageous Effects of Invention

The present invention can solve the various problems in the art and achieve the above object, and can provide: an evaluation method that can highly accurately evaluate whether a subject suffers from depression or whether a subject has possibility of development of depression; an evaluation method that can highly accurately evaluate whether the therapy for depression implemented to the patient who has already suffered from depression is effective; and a method for acquiring data for highly accurately evaluating whether a subject suffers from depression or whether a subject has possibility of development of depression, or whether the therapy for depression implemented to the patient who has already suffered from depression is effective.

### Brief Description of Drawings

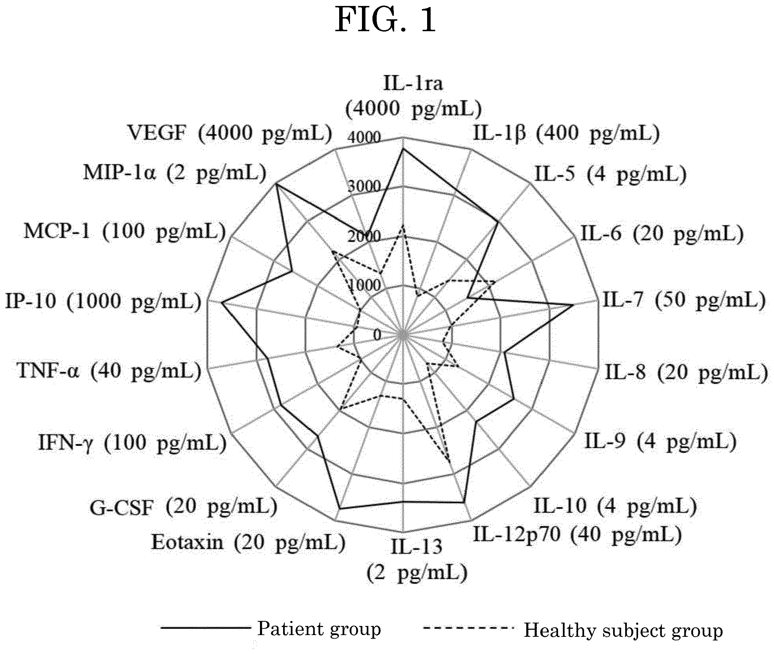

FIG. 1 is a graph indicating average values of concentrations of respective 18 kinds of cytokines contained in saliva analyzed in Analysis 3 of Example 1. The solid line indicates average values of concentrations of respective cytokines contained in saliva sampled from a group of patients with depression (n=20) and the dashed line indicates average values of concentrations of respective cytokines contained in saliva sampled from a group of healthy subjects (n=20).

### Description of Embodiments

### (Evaluation method of depression)

The method of the present invention for evaluating whether an evaluation subject suffers from depression or whether an evaluation subject has possibility of development of depression (hereinafter this method may be referred to as an "evaluation method of depression") includes an evaluation step and if necessary, further includes other steps such as a measurement step.

Depression is one kind of mood disorders. It is a disorder causing, for example, feelings of helplessness and anxiety, characterized by decreased attention, depressive symptoms, loss of vitality, loss of interest, change in appetite (mainly decreases but may increase), change in sleep state, persistent feelings of, for example, sadness and anxiety, and symptoms such as declines in physical functions and mental functions.

### <Evaluation step>

The evaluation step in the evaluation method of depression is a step of evaluating whether the evaluation subject suffers from depression or whether the evaluation subject has possibility of development of depression by using as an indicator an amount (hereinafter may be referred to as a "concentration") of a cytokine in a sample derived from the evaluation subject.

Preferably, the evaluation step further includes using as an indicator an amount of cortisol in the sample derived from the evaluation subject.

In the present invention, whether the evaluation subject suffers from depression also includes the extent of symptoms of depression.

The amounts of the cytokine and the cortisol in the sample derived from the evaluation subject may be those measured in a measurement step described below, or may be those measured separately.

The units of data of the amounts of the cytokine and the cortisol are not particularly limited and may be appropriately selected depending on the intended purpose. Examples of the units include a molar concentration, a weight concentration, and those obtained by adding, subtracting, multiplying, or dividing these concentrations with any coefficient.

### -Evaluation subject-

The evaluation subject is not particularly limited and may be appropriately selected depending on the intended purpose as long as it is a subject required of evaluation of whether the evaluation subject suffers from depression or whether the evaluation subject has possibility of development of depression.

The kind of the evaluation subject is not particularly limited and may be appropriately selected depending on the intended purpose. It may be human or an animal other than human.

The sex of the evaluation subject is not particularly limited and may be appropriately selected depending on the intended purpose. It may be male or female.

The age of the evaluation subject is not particularly limited and may be appropriately selected depending on the intended purpose.

### -Sample derived from evaluation subject-

The sample derived from the evaluation subject is not particularly limited. A sample containing, for example, cells, tissues, or parts thereof sampled from the evaluation subject may be appropriately selected depending on the intended purpose. Examples thereof include blood, serum, cerebrospinal fluid, saliva, throat swab, sweat, urine, tear, lymph fluid, semen, ascites, and breast milk. These may be used alone or in combination.

Of these, preferable are blood, saliva, sweat, and urine; more preferable are saliva, sweat, and urine; and particularly preferable is saliva.

Patients with depression often require a long period for recuperation. In order to appropriately make diagnosis from the viewpoint of side effect points of antidepressants, biological samples obtainable in a non-invasive manner, such as saliva, sweat, and urine, are preferable to biological samples obtainable in an invasive manner, such as blood.

A preparation method of the sample (hereinafter may be referred to as a "sampling method") is not particularly limited and may be appropriately selected from known methods.

When the sample is blood, examples of the sampling method include a method of sampling blood with, for example, a typical syringe.

When the sample is saliva, examples of the sampling method include a method of directly sampling saliva and a method using SALIVETTE or a commercially available tool.

A sampling timing of the sample derived from the evaluation subject (hereinafter may be referred to as a "sampling time slot") is not particularly limited and may be appropriately selected depending on the intended purpose. The sampling timing is preferably almost the same as the timing a sample is taken from a comparative subject in the evaluation step. Especially when the sample is saliva, the amounts of the cytokines contained in saliva may have large daily fluctuations. Thus, it is preferable to make the sampling during a constant time slot in one day.

The sample derived from the evaluation subject may be subjected to treatments such as dilution, if necessary.

### -Cytokine-

The cytokine is a generic term of relatively small proteins (from about 5 to 20 kDa) which play an important role in cell signaling.

The cytokine used in the evaluation step is not particularly limited and may be appropriately selected depending on the intended purpose. Examples thereof include: interleukins; lymphokines; chemokines such as RANTES (regulated on activation, normal T cell expressed and secreted); interferons; hematopoietic growth factors such as colony-stimulating factors (CSFs), granulocyte colony-stimulating factors (G-CSFs), and erythropoietin (EPO); cell growth factors such as epidermal growth factors (EGFs), fibroblast growth factors (FGFs), platelet-derived growth factors (PDGFs), hepatocyte growth factors (HGFs), and transforming growth factors (TGFs); cytotoxic factors such as tumor necrosis factors (TNF-α) and lymphotoxins (TNF-β): adipokines such as leptin and TNF-α: and neurotrophic factors such as nerve growth factors (NGFs).

Of the above cytokines, from the viewpoint of being able to further increase accuracy of evaluation, preferable are one or more kinds selected from the group consisting of interleukin-2 (IL-2), interleukin-4 (IL-4), interleukin-15 (IL-15), interleukin-17A (IL-17A), FGFs, granulocyte-macrophage colony-stimulating factors (GM-CSFs), platelet-derived growth factors (PDGF-BB), macrophage inflammatory protein 1β (MIP-1β), RANTES, interleukin-1 receptor antagonist protein (IL-1ra), interleukin-5 (IL-5), interleukin-10 (IL-10), interleukin-13 (IL-13), granulocyte colony-stimulating factors (G-CSFs), vascular endothelial growth factors (VEGFs), interleukin-1β (IL-1β), interleukin-6 (IL-6), interleukin-7 (IL-7), interleukin-8 (IL-8), interleukin-9 (IL-9), interleukin-12 p70 (IL-12 p70), monocyte chemotactic protein-1 (MCP-1), macrophage inflammatory protein 1α (MIP-1α), TNF-α, interferon-y (IFN-y), interferon-inducible protein 10 (IP-10), and Eotaxin; more preferable are one or more kinds selected from the group consisting of IL-1ra, IL-5, IL-10, IL-13, G-CSF, VEGF, IL-1β, IL-6, IL-7, IL-8, IL-9, IL-12 p70, MCP-1, MIP-1α, TNF-α, IFN-γ, IP-10, and Eotaxin; further preferable are one or more kinds selected from the group consisting of IL-1β, IL-7, IL-8, IL-9, IL-12 p70, MCP-1, IP-10, MIP-1α, TNF-α, IFN-γ, and Eotaxin; further more preferable are one or more kinds selected from the group consisting of IL-7, IL-9, IP-10, and IL-1β; and particularly preferable are one or more kinds selected from the group consisting of IL-7 and IL-9.

The above cytokines may be used alone. From the viewpoint of being able to further increase accuracy of evaluation, however, use of two or more kinds thereof is preferable.

When two or more kinds of the cytokines are used in combination, combinations thereof are not particularly limited and may be appropriately selected depending on the intended purpose. From the viewpoint of being able to further increase accuracy of evaluation, preferable are combinations of two or more kinds of the cytokines except for IL-6; more preferable are combinations of two or more kinds selected from the group consisting of IL-7, IL-9, IP-10, and IL-1β; further preferable are combinations of IL-7 or IL-9 and two or more kinds selected from the cytokines except for IL-7 and IL-9; and particularly preferable is a combination of IL-7 and IL-9.

### -Cortisol-

The cortisol is one kind of glucocorticoids which are corticosteroids. It is an essential hormone to living organisms that control metabolism of hydrocarbons, lipids, and proteins. The cortisol is a hormone synthesized by cells that produce adrenocorticotropic hormone (ACTH) produced at the pituitary gland by the action of corticotropin-releasing hormone (CRH) released from the hypothalamus (diencephalon). Its known effects are, for example, an effect for elevating the blood glucose level through, for example, promotion of gluconeogenesis, an anti-stress effect, an anti-inflammatory effect, an effect to cognition and emotion, an effect of metabolizing calcium *in vivo,* and an effect of promoting phospholipid synthesis. Excessive secretion of cortisol causes, for example, Cushing syndrome which is a disease accompanying muscle weakness and abnormal glucose tolerance. Conversely, reduced secretion of cortisol causes, for example, systemic muscle weakness and in the worst case, leads to cardiac arrest. Thus, secretion of cortisol *in vivo* is strictly controlled.

### -Combined use of cytokine and cortisol-

In the evaluation step, the cytokine may be used alone. From the viewpoint of being able to further increase accuracy of evaluation, however, the cytokine is preferably used in combination with the cortisol.

When the cytokine and the cortisol are used in combination, examples of the cytokine used include those similar to the cytokines described in the above section - Cytokine- and preferable aspects thereof are also similar.

### - Evaluation-

A method of the evaluation is not particularly limited and may be appropriately selected depending on the intended purpose as long as it can evaluate whether the evaluation subject suffers from depression or whether the evaluation subject has possibility of development of depression.

One exemplary method of the evaluation is a method including a process to compare the amount of the cytokine in the sample derived from the evaluation subject, with a standard value. In the process, furthermore, the amount of the cortisol in the sample derived from the evaluation subject may be compared with a standard value.

The standard value (hereinafter may be referred to as a "threshold") is not particularly limited and may be appropriately selected depending on the intended purpose. Example thereof include (I) a value based on the amount of a cytokine or cortisol in a sample derived from a healthy individual (hereinafter this amount may be referred to as a "normal value") and (II) a value based on the amount of a cytokine or cortisol in a sample derived from a patient with depression (hereinafter this amount may be referred to as a "disease value"). These may be used alone or in combination.

The patient with depression may be a typical patient with depression or may be a patient with mild, moderate, or severe depression.

In general, the threshold in a diagnostic drug (so-called "cut-off value") is appropriately set by persons skilled in the art depending on the intended purpose based on many measurements of healthy subjects and patients obtained in clinical trials. From the matters described in the present specification, persons skilled in the art can easily determine the threshold in the following manner, for example.

The value based on the normal value in the above (I) is not particularly limited and may be appropriately selected depending on the intended purpose. For example, when top priority is given to avoidance of missing a disease group like in a screening test, sensitivity is prioritized to specificity, and with reference to the normal value, the threshold is set to, for example, the normal value or a close value to the normal value.

The value based on the disease value in the above (II) is not particularly limited and may be appropriately selected depending on the intended purpose. For example, when evaluating the extent of symptoms of depression, with reference to the disease value of the patient with mild, moderate, or severe depression, the threshold is set to, for example, the disease value or a close value to the disease value.

In the process, when the standard value is the value based on the normal value in the above (I), the evaluation subject can be evaluated to suffer from depression or have possibility of development of depression when the amount of the cytokine or cortisol in the sample derived from the evaluation subject is more than the value based on the normal value.

The healthy individual is an individual of the same kind as the evaluation subject and is preferably an individual with a close age to the evaluation subject.

When the standard value is the value based on the disease value in the above (II), the evaluation subject can be evaluated to suffer from depression or have possibility of development of depression when the amount of the cytokine or cortisol in the sample derived from the evaluation subject is equal to or more than the value based on the disease value.

The patient with depression is an individual of the same kind as the evaluation subject and is preferably a patient with a close age to the evaluation subject.

In the evaluation step, regardless of the concentration of IL-6 in the sample, highly accurate evaluation can be made using at least one kind of the cytokines except for IL-6. IL-6 is a cytokine that is known to increase in the blood concentration at the acute stage of depression. The IL-6 concentration also increases in the blood of patients with autoimmune diseases such as rheumatism. Therefore, use of the present invention makes it possible to evaluate whether a subject suffers from depression or whether a subject has possibility of development of depression, also for patients whose depressive symptoms are not alleviated but whose IL-6 concentration decreases as a result of administration of other drugs such as rheumatism-related drugs, for patients having continuing depressive symptoms not being at the acute stage of depression, or for patients who are highly likely to assume depressive symptoms.

In the evaluation step, for example, the fact that "the concentration of the cytokine or cortisol is high" can be judged based on whether the concentration of the cytokine or cortisol in the sample derived from the evaluation subject is higher than the threshold (for example, the value based on the concentration of the cytokine or cortisol in the sample derived from the healthy individual). The fact that "the concentration of the cytokine or cortisol is low" can be judged based on whether the concentration of the cytokine or cortisol in the sample derived from the evaluation subject is lower than the threshold.

The standard value used may be set based on the amount of the cytokine or cortisol in the sample measured when implementing the evaluation method of depression, or may be set based on the amount of the cytokine or cortisol in the sample that has already been measured.

### --Evaluation formula--

The method of evaluation may also be a method using an evaluation formula that can evaluate whether the evaluation subject suffers from depression or whether the evaluation subject has possibility of development of depression.

In one exemplary method thereof, based on data of the amount of the cytokine and if necessary, based on data of the amount of the cortisol, the data of the amount of the cytokine and if necessary, the data of the amount of the cortisol are substituted into an evaluation formula that can evaluate whether the evaluation subject suffers from depression or whether the evaluation subject has possibility of development of depression, to evaluate whether the evaluation subject suffers from depression or whether the evaluation subject has possibility of development of depression.

Here, the evaluation formula includes variables into which the data of the amount of the cytokine (hereinafter may be referred to as a "concentration value of the cytokine") and if necessary, the data of the amount of the cortisol (hereinafter may be referred to as a "concentration value of the cortisol) are substituted.

The number of the variables is not particularly limited and may be appropriately selected depending on the intended purpose as long as it is at least one. From the viewpoint of being able to further increase accuracy of evaluation, the number of the variables is preferably at least two. That is, it is preferable to include substituting the data of the amounts of two or more kinds of substances selected from the group consisting of the cytokines and the cortisol into the evaluation formula that can evaluate whether the evaluation subject suffers from depression or whether the evaluation subject has possibility of development of depression based on the data of the amounts of two or more kinds of substances selected from the group consisting of the cytokines and the cortisol, with the evaluation formula including at least two variables into which the data of the amounts of two or more kinds of substances selected from the group consisting of the cytokines and the cortisol are substituted.

An aspect of two or more kinds of substances selected from the group consisting of the cytokines and the cortisol is not particularly limited and may be appropriately selected depending on the intended purpose. Examples of the aspect include an aspect where at least one kind of the cytokines and the cortisol are used in combination and an aspect where at least two kinds of the cytokines are used.

The evaluation formula is not particularly limited and may be appropriately selected depending on the intended purpose. Examples thereof include a logistic regression formula, a fractional formula, a linear discriminant, a multiple regression formula, a formula created by a support vector machine, a formula created by the Mahalanobis distance method, a formula created by canonical discriminant analysis, and a formula created by decision trees. Use of them can realize further increase in reliability of information that can be referred to for knowing the state of depression.

A formula used as the evaluation formula refers to the format of a formula generally used for multivariate analyses. Examples thereof include a fractional formula, a multiple regression formula, a multiple logistic regression formula, a linear discriminant function, a Mahalanobis distance, a canonical discriminant function, a support vector machine, decision trees, and a formula expressed with the sum of formulae having different formats. Here, for a multiple regression formula, a multiple logistic regression formula, and a canonical discriminant function, a coefficient and a constant term are added to each variable. Preferably, these coefficient and constant term may be real numbers. More preferably, they may be values within the ranges of 99% confidential intervals of the coefficient and constant term obtained from the data for making the above various classifications. Further preferably, they may be values within the ranges of 95% confidential intervals of the coefficient and constant term obtained from the data for making the above various classifications. The value of each coefficient and its confidential interval may be those obtained by multiplying them with real numbers. The value of the constant term and its confidential interval may be those obtained by adding, subtracting, multiplying, and dividing them with any real constant. When a display formula such as logistic regression, linear discriminant, or multiple regression analysis is used as the evaluation formula, the display formula also includes those after linear transformation of the display formula (addition of a constant, multiplication with a constant) and transformation of monotonically increasing (decreasing) (for example, logit transformation). This is because these transformations do not change evaluation performances, and the display formula is comparable between before and after the transformations.

For example, as a risk calculation formula, a formula of (the concentration of cytokine A)+(the concentration of cytokine B) is exemplified when evaluation is made using the concentration data of two kinds of cytokines. A formula of (the concentration of cytokine A)+(the concentration of cytokine B)+(the concentration of cortisol) is exemplified when evaluation is made using the concentration data of two kinds of cytokines and the concentration data of cortisol. According to this, the evaluation of whether the evaluation subject suffers from depression or whether the evaluation subject has possibility of development of depression can be quantified as a specific numerical value. Also, based on the results obtained by studying the correlations of various cytokines and cortisol with patients with depression, weighting may be performed in advance for cortisol and the kinds of cytokines. Moreover, the evaluation may be made based on a value obtained by adding (the value of concentration data of each cytokine and/or cortisol of an evaluation subject)/(the average value of concentration data of each cytokine and/or cortisol of a healthy subject) (or after the obtained value is weighted to a certain extent). Further, depending on, for example, the age, sex, race, and presence or absence of genetic background of depression, the value of the cytokine concentration and/or the cortisol concentration may be corrected. The extent of the correction or the presence or absence of the correction may be changed depending on the cortisol or the kinds of the cytokines. The value of the evaluation obtained in the above-described manner may be compared with the corresponding value of a typical person (for example, the average value of Japanese) to evaluate whether the evaluation subject suffers from depression or whether the evaluation subject has possibility of development of depression based on the fold change therebetween.

Examples of the cytokines and cortisol in the method using the evaluation formula include those similar to the cytokines and cortisol described in the above sections -Cytokine-, -Cortisol-, and -Combined use of cytokine and cortisol- and preferable aspects thereof are also similar.

In extracting the cytokine and if necessary, the cortisol, for example, the AUC (Area Under the Curve) of a ROC (Receiver Operating Characteristic curve) can be used. In the present invention, it is possible to use, for example, backward elimination to determine the combination of the cytokine and if necessary, the cortisol that gives the largest AUC.

### <Other steps>

The other steps in the evaluation method of depression are not particularly limited and may be appropriately selected depending on the intended purpose as long as they do not degrade the effects of the present invention. Examples thereof include a measurement step and a sample preparation step.

### «Measurement step»

The measurement step in the evaluation method of depression is a step of measuring the amount of the cytokine in the sample derived from the evaluation subject.

Preferably, the measurement step further includes measuring the amount of the cortisol in the sample derived from the evaluation subject.

Examples of the sample, cytokine, and cortisol in the measurement step include those similar to the sample, cytokine, and cortisol described in the above section <Evaluation step> and preferable aspects thereof are also similar.

The measurement step may be performed in combination with measurement of the amount of a substance other than the cytokine and cortisol.

### -Measurement-

A method for measuring the amount of the cytokine in the sample is not particularly limited and may be appropriately selected from known methods. Examples of the method include a method of measuring the amount of a protein and a method of measuring the amount of mRNA. These may be used alone or in combination.

The amino acid sequence or base sequence of the cytokine is easily available via public databases such as GenBank (NCBI).

The method of measuring the amount of the protein of the cytokine is not particularly limited and may be appropriately selected from known methods. Examples thereof include a method of measuring it according to an immunological technique using an antibody that specifically recognizes the cytokine.

The immunological technique is not particularly limited and may be appropriately selected from known methods. Examples thereof include an antibody array, flow cytometry analysis, radioimmunoassay (RIA), ELISA (Methods in Enzymol. 70: 419-439 (1980)), dot blotting, immunonucleic acid assay, Western blotting, and immunohistological staining.

Specific examples of the ELISA include: the direct method in which a target cytokine is immobilized on a microplate and a labelled antibody that recognizes the cytokine is acted thereon; the indirect method using a labelled secondary antibody that recognizes an anti-cytokine antibody; the sandwich method in which an anti-cytokine antibody is immobilized and a labelled anti-cytokine antibody is further used; and the competitive method in which an anti-cytokine antibody is immobilized and a target protein and an enzyme-labelled antigen having a known concentration are allowed to react at the same time in the same microplate.

The method of measuring the amount of the mRNA is not particularly limited and may be appropriately selected from known methods. Examples thereof include PCR, real-time PCR, a DNA array method, and Northern blotting.

The amount of the cytokine in the sample can also be measured using a commercially available measurement kit for the amount of a cytokine.

The method for measuring the amount of the cortisol in the sample is not particularly limited and may be appropriately selected from known methods. Examples thereof include a method of measuring it according to an immunological technique using an antibody that specifically recognizes the cortisol.

The amount of the cortisol in the sample can also be measured using a commercially available measurement kit for the amount of cortisol.

### «Sample preparation step»

The sample preparation step in the evaluation method of depression is a step of preparing a sample derived from the evaluation subject, and may be appropriately selected from known methods depending on the kind of the sample.

The evaluation method of depression of the present invention can also be performed in combination with evaluation by other means such as interviews.

According to the evaluation method of depression of the present invention, it is possible to highly accurately evaluate whether the evaluation subject suffers from depression or whether the evaluation subject has possibility of development of depression by using as an indicator the amount of the cytokine and if necessary, the amount of the cortisol in the sample derived from the evaluation subject.

Therefore, the present invention also relates to: a method for diagnosing whether an evaluation subject suffers from depression or whether an evaluation subject has possibility of development of depression, the method including the evaluation step and if necessary, further including the other steps; or a method for aiding diagnosis of whether an evaluation subject suffers from depression or whether an evaluation subject has possibility of development of depression.

Further, the present invention relates to a biomarker for evaluating, diagnosing, or diagnosis-aiding whether an evaluation subject suffers from depression or whether an evaluation subject has possibility of development of depression, the biomarker being formed of a cytokine and if necessary, cortisol.

### (Evaluation method of the effect of the therapy for depression)

The method of the present invention for evaluating the effect of the therapy for depression in the evaluation subject (hereinafter may be referred to as an "evaluation method of the effect of the therapy for depression") includes an evaluation step and if necessary, further includes other steps such as a measurement step.

### <Evaluation step>

The evaluation step in the evaluation method of the effect of the therapy for depression is a step of evaluating the effect of the therapy for depression implemented to the evaluation subject (whether the therapy is effective) by using as an indicator the amount (hereinafter may be referred to as a "concentration") of the cytokine in the sample derived from the evaluation subject.

Preferably, the evaluation step further includes using as an indicator the amount of cortisol in the sample derived from the evaluation subject.

In the present invention, the evaluation of the effect of the therapy for depression also includes the extent of the effect of the therapy.

The therapy for depression is not particularly limited and may be appropriately selected depending on the intended purpose. Examples thereof include behavior therapy, drug therapy, and cognitive therapy.

The amounts of the cytokine and the cortisol in the sample derived from the evaluation subject may be those measured in a measurement step described below, or may be those measured separately.

The units of data of the amounts of the cytokine and the cortisol are not particularly limited and may be appropriately selected depending on the intended purpose. Examples of the units include a molar concentration, a weight concentration, and those obtained by adding, subtracting, multiplying, or dividing these concentrations with any coefficient.

### -Evaluation subject-

The evaluation subject is not particularly limited and may be appropriately selected depending on the intended purpose as long as it is a subject to which the therapy for depression has been implemented.

The kind of the evaluation subject is not particularly limited and may be appropriately selected depending on the intended purpose. It may be human or an animal other than human.

The sex of the evaluation subject is not particularly limited and may be appropriately selected depending on the intended purpose. It may be male or female.

The age of the evaluation subject is not particularly limited and may be appropriately selected depending on the intended purpose.

### -Sample derived from evaluation subject-

The sample derived from the evaluation subject is not particularly limited. Examples thereof include those similar to the samples described in the above section -Sample derived from evaluation subject- (of the evaluation method of depression) and preferable aspects thereof are also similar.

In the evaluation method of the effect of the therapy for depression, the sample is preferably taken from the evaluation subject before and after implementation of the therapy of depression.

A preparation method and sampling time slot of the sample are not particularly limited. Examples of the preparation method and sampling time slot include those similar to the preparation methods and sampling time slots described in the above section -Sample derived from evaluation subject- (of the evaluation method of depression) and preferable aspects thereof are also similar.

The sample may be subjected to treatments such as dilution, if necessary.

### -Cytokine-

The cytokine is not particularly limited. Examples thereof include those similar to the cytokines in the above section -Cytokine- (of the evaluation method of depression) and preferable aspects thereof are also similar.

### -Cortisol-

The cortisol is similar to that described in the above section -Cortisol- (of the evaluation method of depression).

### -Combined use of cytokine and cortisol-

In the evaluation step, the cytokine may be used alone. From the viewpoint of being able to further increase accuracy of evaluation, however, the cytokine is preferably used in combination with the cortisol.

When the cytokine and the cortisol are used in combination, examples of the cytokine used include those similar to the cytokines described in the above section - Cytokine- (of the evaluation method of depression) and preferable aspects thereof are also similar.

### - Evaluation-

A method of the evaluation is not particularly limited and may be appropriately selected depending on the intended purpose as long as it can evaluate the effect of the therapy for depression in the evaluation subject.

One exemplary method of the evaluation is a method including a process to compare the amount of the cytokine in the sample derived from the evaluation subject, with a reference value. In the process, furthermore, the amount of the cortisol in the sample derived from the evaluation subject may be compared with a reference value.

The reference value is not particularly limited and may be appropriately selected depending on the intended purpose. Example thereof include (i) a value based on the amount of a cytokine or cortisol in a sample derived from the evaluation subject and obtained before implementation of the therapy for depression (hereinafter this amount may be referred to as a "disease value before the therapy") and (ii) a value based on the amount of a cytokine or cortisol in a sample derived from a patient with depression (hereinafter this amount may be referred to as a "disease value"). These may be used alone or in combination.

The patient with depression may be a typical patient with depression or may be a patient with mild, moderate, or severe depression.

From the matters described in the present specification, persons skilled in the art can easily determine the reference value in the following manner, for example.

The value based on the disease value before the therapy in the above (i) is not particularly limited and may be appropriately selected depending on the intended purpose. For example, with reference to the disease value before the therapy in the above (i), the value based on the disease value before the therapy is set to, for example, the disease value before the therapy or a close value to the disease value before the therapy.

The value based on the disease value in the above (ii) is not particularly limited and may be appropriately selected depending on the intended purpose. For example, when evaluating the extent of the effect of the therapy for depression, with reference to the disease value of the patient with mild, moderate, or severe depression, the threshold is set to, for example, the disease value or a close value to the disease value.

In the process, when the reference value is the value based on the disease value before the therapy in the above (i), the effect of the therapy for depression in the evaluation subject can be evaluated to be low when the amount of the cytokine or cortisol in the sample derived from the evaluation subject is equal to or more than the value based on the disease value before the therapy. The expression of "the effect... low" also includes "the effect ... none".

When the reference value is the value based on the disease value in the above (ii), the effect of the therapy for depression in the evaluation subject can be evaluated to be low when the amount of the cytokine or cortisol in the sample derived from the evaluation subject is equal to or more than the value based on the disease value. The expression of "the effect... low" also includes "the effect... none".

The patient with depression is an individual of the same kind as the evaluation subject and is preferably a patient with a close age to the evaluation subject.

In the evaluation step, regardless of the concentration of IL-6 in the sample, highly accurate evaluation can be made using at least one kind of the cytokines except for IL-6. IL-6 is a cytokine that is known to increase in the blood concentration at the acute stage of depression. The IL-6 concentration also increases in the blood of patients with autoimmune diseases such as rheumatism. Therefore, use of the present invention makes it possible to evaluate the effect of the therapy for depression, also for patients whose depressive symptoms are not alleviated but whose IL-6 concentration decreases as a result of administration of other drugs such as rheumatism-related drugs, for patients having continuing depressive symptoms not being at the acute stage of depression, or for patients who are highly likely to assume depressive symptoms.

In the evaluation step, for example, the fact that "the concentration of the cytokine or cortisol is high" can be judged based on whether the concentration of the cytokine or cortisol in the sample derived from the evaluation subject is higher than the reference value (for example, the value based on the concentration of the cytokine or cortisol in the sample derived from the evaluation subject and obtained before implementation of the therapy for depression). The fact that "the concentration of the cytokine or cortisol is low" can be judged based on whether the concentration of the cytokine or cortisol in the sample derived from the evaluation subject is lower than the reference value.

When the reference value is the value based on the amount of the cytokine or cortisol in the sample derived from the patient with depression in the above (ii), the reference value used may be set based on the amount of the cytokine or cortisol in the sample measured when implementing the evaluation method of the effect of the therapy for depression, or may be set based on the amount of the cytokine or cortisol in the sample that has already been measured.

### --Evaluation formula--

The method of evaluation may also be a method using an evaluation formula that can evaluate the effect of the therapy for depression.

The method using the evaluation formula is the same as the above evaluation method of depression using the evaluation formula, except that in the section --Evaluation formula-- of -Evaluation- of the above (Evaluation method of depression), the evaluating whether the evaluation subject suffers from depression or whether the evaluation subject has possibility of development of depression is changed to evaluating the effect of the therapy for depression, and preferable aspects thereof are also similar.

### <Other steps>

The other steps in the evaluation method of the effect of the therapy for depression are not particularly limited and may be appropriately selected depending on the intended purpose as long as they do not degrade the effects of the present invention. Examples thereof include those similar to the other steps described in the above section <Other steps> of the (Evaluation method of depression).

The evaluation method of the effect of the therapy for depression of the present invention can also be performed in combination with evaluation by other means such as interviews.

According to the evaluation method of the effect of the therapy for depression, it is possible to highly accurately evaluate whether the therapy for depression in the evaluation subject is effective by using as an indicator the amount of the cytokine and if necessary, the amount of the cortisol in the sample derived from the evaluation subject.

Accordingly, the present invention also relates to a method for diagnosing whether a therapy for depression is effective in an evaluation subject or a method for aiding diagnosis of whether a therapy for depression is effective in an evaluation subject, each of the methods including the evaluation step and if necessary further including the other steps.

Also, the present invention relates to a biomarker for evaluating, diagnosing, or diagnosis-aiding whether a therapy for depression is effective in an evaluation subject, the biomarker being formed of a cytokine and if necessary, cortisol.

### (Method for acquiring data for evaluation)

The method of the present invention for acquiring data for evaluating whether the evaluation subject suffers from depression or whether the evaluation subject has possibility of development of depression, or data for evaluating the effect of the therapy for depression in the evaluation subject (hereinafter may be referred to as an "evaluation data acquisition method") includes a measurement step and if necessary, further includes other steps.

### <Measurement step>

The measurement step in the evaluation data acquisition method is a step of measuring the amount of a cytokine in a sample derived from the evaluation subject. The measurement step can be performed in the same manner as in the section «Measurement step» described above in the section of the other steps of the evaluation method of depression of the present invention, and preferable aspects thereof are also similar.

### <Other steps>

The other steps in the evaluation data acquisition method are not particularly limited and may be appropriately selected depending on the intended purpose as long as they do not degrade the effects of the present invention. Examples thereof include a sample preparation step.

### «Sample preparation step»

The sample preparation step in the evaluation data acquisition method is a step of preparing the sample derived from the evaluation subject. The sample preparation step can be performed in the same manner as in «Sample preparation step» described above in the section of the other steps of the evaluation method of depression of the present invention.

Preferably, the evaluation data acquisition method of the present invention further includes measuring the amount of cortisol in the sample derived from the evaluation subject.

The evaluation data acquisition method may be performed in combination with measurement of the amount of a substance other than the cytokine and the cortisol.

The evaluation data acquisition method of the present invention can collect data useful for highly accurately evaluating whether the evaluation subject suffers from depression or whether the evaluation subject has possibility of development of depression, or whether the therapy for depression is effective in the evaluation subject.

### (Kit for evaluating, diagnosing, and diagnosis-aiding depression or the effect of a therapy for depression)

As described above, the evaluation method of depression of the present invention can highly accurately evaluate whether the evaluation subject suffers from depression or whether the evaluation subject has possibility of development of depression by using as an indicator the amount of the cytokine and if necessary, the amount of the cortisol in the sample derived from the evaluation subject. Also, the evaluation method of the effect of a therapy for depression of the present invention can high accurately evaluate whether the therapy for depression is effective in the evaluation subject by using as an indicator the amount of the cytokine and if necessary, the amount of the cortisol in the sample derived from the evaluation subject. Accordingly, the present invention also relates to a kit for evaluating, diagnosing, and diagnosis-aiding whether the evaluation subject suffers from depression or whether the evaluation subject has possibility of development of depression, or whether the therapy for depression is effective in the evaluation subject (hereinafter this kit may be referred to as a "kit for evaluating, diagnosing, and diagnosis-aiding depression or the effect of a therapy for depression").

The kit for evaluating, diagnosing, and diagnosis-aiding depression or the effect of a therapy for depression includes a cytokine detection unit and if necessary further includes other elements such as a cortisol detection unit.

### <Cytokine detection unit>

The cytokine detection unit is not particularly limited and may be appropriately selected depending on the intended purpose as long as it can detect the target cytokine in the sample. In order to immunologically detect the cytokine, the cytokine detection unit preferably includes a detection tool on which an antibody against the cytokine has been immobilized.

### <Cortisol detection unit>

The cortisol detection unit is not particularly limited and may be appropriately selected depending on the intended purpose as long as it can detect cortisol in the sample. In order to immunologically detect the cortisol, the cortisol detection unit preferably includes a detection tool on which an antibody against the cortisol has been immobilized.

Examples of the cytokine and cortisol include those similar to the cytokines and cortisol described in the section -Cytokine- and the section -Cortisol- in the

### <Evaluation step> in the evaluation method of depression of the present invention, and preferable aspects thereof are also similar.

An object on which an antibody against the cytokine or cortisol is to be immobilized is not particularly limited and may be appropriately selected depending on the intended purpose as long as the object is a carrier that is a solid or an insoluble material. Examples thereof include carriers that can be separated from a reaction mixture through, for example, filtration, precipitation, or magnetic separation.

The shape of the carrier is not particularly limited and may be appropriately selected depending on the intended purpose. Examples thereof include beads, magnetic beads, thin films, micro-tubes, filters, plates, microplates, carbon nanotubes, and sensor chips. Flat carriers such as the thin film and the plate may be provided with, for example, a pit, a groove, or a filter bottom.

### <Other elements>

The other elements are not particularly limited and may be appropriately selected depending on the intended purpose from the elements included in, for example, known diagnosis kits. Examples thereof include reagents used for an immunological measurement method and written instructions for the kit.

According to the kit for evaluating, diagnosing, and diagnosis-aiding depression or the effect of a therapy for depression, it is possible to highly accurately evaluate, diagnose, and diagnosis-aid whether the evaluation subject suffers from depression or whether the evaluation subject has possibility of development of depression, or whether the therapy for depression is effective in the evaluation subject.

### Examples

The present invention will be described below by way of Examples. However, the present invention should not be construed as being limited to these Examples.

### (Example 1)

Test subjects used were two groups of 20 patients with depression (a patient group) and 35 healthy subjects (a healthy subject group). The patient group was gathered before the healthy subject group, and the healthy subjects were gathered afterwards who matched the patient group in terms of the number of the subjects, the male-to-female ratio, and the average age.

The patient group was composed of adult patients who met the diagnosis criteria of the major depressive disorder (7 to 20 points according to the Hamilton depression rating scale) and were receiving behavior therapy.

The healthy subject group was composed of subjects who did not fall under all of the following items (1) to (5):
(1) suffering from any of an inflammatory disease, an endocrine disease, a metabolic disease, and a severe periodontal disease;
(2) taking any of an anti-allergic agent, a steroidal agent, and an immunosuppressive agent within one month;
(3) having a cerebral organic disorder;
(4) having a past history of suffering from a psychogenic mental disorder, an endogenous mental disorder, and an organic mental disorder other than the major depressive disorder; and
(5) having a habit of smoking.

A saliva sample was taken once one hour after they got up in the early morning. The taken saliva was analyzed for cytokines (27 kinds) and cortisol contained therein. Fifteen out of the 35 healthy subjects of the healthy subject group were excluded from the analysis because they had a lot of data loss.

Table 1 indicates the backgrounds of the test subjects.

**Table 1**

| Test subjects | Number | Sex | | Average age | Oldest | Youngest | Average age of males | Average age of females |
|---|---|---|---|---|---|---|---|---|
| | | Male | Female | | | | | |
| Patient group | 20 | 13 | 7 | 51.3±16.5 | 77 | 23 | 50.2 | 53.3 |
| Healthy subject group | 20 | 13 | 7 | 51.3±3.7 | 57 | 43 | 50.2 | 53.3 |

The following gives a sample collection organization, an analyzer, a cytokine analysis kit, kinds of cytokines analyzed, a cortisol analysis kit, and details of a testing method.
- Sample collection organization: Shinshu University Hospital
- Cytokine analyzer: Bio-Plex (Bio-Rad Laboratories, Inc.)
- Cytokine analysis kit: Bio-Plex Pro Human Cytokine 27-Plex (Bio-Rad Laboratories, Inc.)
- Kinds of cytokines analyzed:
   IL-1β, IL-1ra, IL-2, IL-4, IL-5, IL-6, IL-7, IL-8, IL-9, IL-10, IL-12 p70, IL-13, IL-15, IL-17A, FGF, Eotaxin, G-CSF, GM-CSF, IFN-γ, IP-10, MCP-1, MIP-1α, PDGF-BB, MIP-1β, RANTES, TNF-α, VEGF
- Cortisol analyzer: Fluorescent microplate reader ARVO MX (Perkin Elmer INC.)
- Cortisol analysis kit: Cortisol Salivary immunoassay Kit (Salimetrics LLC)
- Testing method:
   (I) Sampling method of measurement samples from saliva
      i) Two sampling cups (Japan Medical Corporation: Model No. MS-50) were provided and written with ID (the two sampling cups are given the same ID).
      ii) A line is drawn at a level of 200 µL of each of the sampling cups.
      iii) A necessary amount (about 200 µL) of saliva is put into each of the two cups, followed by cryopreservation at -80°C within two hours after the sampling.

Each of the samples obtained in the above manner was measured for the concentrations of 27 kinds of cytokines and cortisol by using the above-described two kinds of analyzers and analysis kits.

The obtained analysis results were tested by the Mann-Whitney test using statistical analysis software (IBM SPSS Statistics, IBM Japan, Ltd.).

When the measured concentration values of the cytokines and cortisol are lower than the limit of detection but only when a value of LOD (Limit of Detection) can be calculated, a value of LOD/2 was used instead as the measurement value (where the LOD refers to a concentration obtained by adding a concentration fluctuation observed between blanks to the concentration of a blank, meaning the measurable lower-limit value calculated from the blank; the calculation formula being as follows: the value of a blank + 2 × the SD value of the blank). Conditions under which this is applicable are only when the LOD/2 value is a lower value than the sample concentration and the measurable lower-limit value of LLOQ (a lower concentration range of a standard liquid). When the LLOQ value was lower than the LOD/2 value, the LLOQ/2 value was used as the measurement value.

The LOD value was calculated using the coefficients of a standard curve regression formula determined from the actually measured values of eight standard concentrations. When the LOD/2 value is higher than the lowest concentration value (S8) of the standards, the S8 concentration value/2 was used as the measurement value.

### <Analysis 1>

The concentrations of the cytokines in the saliva taken from the patient group were compared with the concentrations of the cytokines in the saliva taken from the healthy subject group.

### -Analysis result 1-

Except for IL-6, the concentrations of the cytokines in the saliva taken from the patient group were detected to be higher than the concentrations of the cytokines in the saliva taken from the healthy subject group.

### <Analysis 2>

The concentration of the cortisol in the saliva taken from the patient group was compared with the concentration of the cortisol in the saliva taken from the healthy subject group.

### -Analysis result 2-

The average value of the cortisol concentrations in the saliva taken from the patient group was found to be 2.58±1.20 ng/mL, while the average value of the cortisol concentrations in the saliva taken from the healthy subject group was found to be 1.43±0.62 ng/mL. This indicates that the cortisol concentration in the saliva is higher in the patient group than in the healthy subjects.

### <Analysis 3>

Statistical analysis by the Mann-Whitney Test was performed on the concentrations of the cytokines and the cortisol concentrations in the saliva of the patient group and on the concentrations of the cytokines and the cortisol concentrations in the saliva of the healthy subject group. Results are presented in Table 2.

Out of the analyzed 27 kinds of cytokines, 9 kinds of cytokines (IL-2, IL-4, IL-15, IL-17A, FGF, GM-CSF, PDGF-BB, MIP-1β, and RANTES) were excluded from the analysis because they had data loss.

### -Analysis result 3-

As a result of Analysis 3, a significant difference was found between the patients and the healthy subjects about the concentrations of some of the cytokines in the saliva (Table 2).

As presented in Table 2, among the cytokines contained in the saliva taken from the patients, IL-1β, IL-7, IL-8, IL-9, IL-12 p70, MCP-1, MIP-1α, TNF-α, IFN-γ, and Eotaxin are indicated to have significantly higher concentrations than the concentrations of those cytokines contained in the saliva taken from the healthy subjects, regardless of their sex and age. Meanwhile, for IL-6, no significant difference was found between the patients and the healthy subjects about the concentration thereof in the saliva. The IL-6 concentration in the saliva was found to be the same extent therebetween or rather be lower in the patients. This is different from the phenomenon that has been reported so far (the IL-6 concentration is significantly higher in the blood of patients with depression), suggesting that the kinetics of the cytokine concentrations in saliva and blood are different between patients and healthy subjects. The patient group in the present analysis was directed to patients with moderate depressive symptoms rather than patients at the acute stage of depression. Therefore, even in the case of a patient who is not evaluated to suffer from depression according to the conventional evaluation of depression using IL-6 in the blood but actually shows depressive symptoms, the possibility of being able to evaluate depression was shown; i.e., the evaluation of depression could be successfully made regardless of the stage of depression.

A graph indicating the average values of the concentrations of the analyzed respective 18 kinds of cytokines contained in saliva is presented in FIG. 1 (the solid line: the patient group, the dashed line: the healthy subject group).

Regarding the concentration of cortisol in the saliva of the patients and the healthy subjects, the concentration of cortisol in the saliva of the patients was indicated to be significantly higher than the concentration of cortisol in the saliva of the healthy subjects.

**Table 2**

| Significant difference was found between patient group and healthy subject group (p value < 0.05) | | No significant difference was found between patient group and healthy subject group (p value ≥ 0.05) |
|---|---|---|
| Patient group < Healthy subject group | Patient group > Healthy subject group | IP-10, IL-10, VEGF, IL-1ra, G-CSF, IL-6, IL-5, IL-13 |
| None | IL-1β, IL-7, IL-8, IL-9, IL-12p70, MCP-1, MIP-1α, TNF-α, IFN-γ, Eotaxin, cortisol | |

### <Analysis 4>

The cytokines and cortisol that had been found to have significant differences between the patient group and the healthy subject group as a result of Analysis 3 were further modeled through logistic regression analysis using the stepwise method and validated for determination capability through ROC analysis.

### -Analysis result 4-

Table 3 presents the results of Analysis 4; i.e., the calculated value of the AUC (Area Under Curve) through ROC analysis in the combinations of the cytokines and cortisol.

**Table 3**

| | Independent variable | ROC analysis | | |
|---|---|---|---|---|
| | | Sensitivity | Specificity | AUC |
| Combinations of cytokines and cortisol | Cortisol | 0.947 | 0.833 | 0.912 |
| | IL-7 | | | |
| | Cortisol | 0.950 | 0.800 | 0.888 |
| | IL-9 | | | |
| | Cortisol | 0.700 | 0.850 | 0.785 |
| | IP-10 | | | |
| | Cortisol | 0.947 | 0.850 | 0.918 |
| | IL-7 | | | |
| | IL-9 | | | |
| Combinations of cytokines | IL-7 | 0.684 | 0.889 | 0.816 |
| | IL-9 | | | |
| | IP-10 | 0.700 | 0.850 | 0.778 |
| | IL-1β | | | |
| Single use | Cortisol | 0.700 | 0.800 | 0.771 |
| | IL-7 | 0.632 | 0.889 | 0.792 |
| | IL-9 | 0.650 | 0.750 | 0.764 |
| | IP-10 | 0.500 | 0.800 | 0.653 |

The obtained results suggest that the combination of cortisol and IL-7, IL-9 or IP-10 is preferable in the evaluation of depression. Also in the combinations of the cytokines alone, the combination of IL-7 and IL-9 is indicated to be preferable. Note that, cortisol, IL-7, or IL-9 exhibits a large AUC even in single use, suggesting the possibility of successful evaluation by single use thereof.

From the above results, it has been found that by using as an indicator the amount of the cytokine contained in the sample derived from the evaluation subject and if necessary, the amount of cortisol, it is possible to highly accurately evaluate whether the evaluation subject suffers from depression or whether the evaluation subject has possibility of suffering from depression, and whether the therapy for depression implemented to the patient who has already suffered from depression is effective.

Also, in view of no significant difference between the patients and the healthy subjects about the IL-6 concentration in the saliva as presented in Analysis 3, it is indicated that regardless of the presence or absence of the difference in the IL-6 concentration from the healthy subjects, use of any of cortisol, IL-7, and IL-9 or combinations thereof makes it possible to evaluate whether the subject suffers from depression or whether the subject has possibility of suffering from depression.

Aspects of the present invention are as follows, for example.
<1> A method for evaluating whether an evaluation subject suffers from depression or whether an evaluation subject has possibility of development of depression, the method including
   evaluating whether the evaluation subject suffers from depression or whether the evaluation subject has possibility of development of depression by using as an indicator an amount of a cytokine in a sample derived from the evaluation subject.
<2> The method according to <1> above, wherein the evaluating includes a process to compare the amount of the cytokine in the sample derived from the evaluation subject, with a standard value,
   the standard value is: a value based on an amount of a cytokine in a sample derived from a healthy individual; a value based on an amount of a cytokine in a sample derived from a patient with depression; or both of the values, and
   the evaluating is evaluating that the evaluation subject suffers from depression or has possibility of development of depression when:
      the amount of the cytokine in the sample derived from the evaluation subject is more than the value based on the amount of the cytokine in the sample derived from the healthy individual;
      the amount of the cytokine in the sample derived from the evaluation subject is equal to or more than the value based on the amount of the cytokine in the sample derived from the patient with depression; or
      the amount of the cytokine in the sample derived from the evaluation subject is more than the value based on the amount of the cytokine in the sample derived from the healthy subject and is equal to or more than the value based on the amount of the cytokine in the sample derived from the patient with depression.
<3> The method according to <1> above, wherein the evaluating includes substituting data of the amount of the cytokine into an evaluation formula that can evaluate, based on the data of the amount of the cytokine, whether the evaluation subject suffers from depression or whether the evaluation subject has possibility of development of depression, and the evaluation formula includes at least one variable into which the data of the amount of the cytokine is substituted.
<4> The method according to any one of <1> to <3> above, wherein the cytokine is one or more kinds of cytokines selected from the group consisting of IL-1β, IL-7, IL-8, IL-9, IL-12 p70, MCP-1, IP-10, MIP-1α, TNF-α, IFN-γ, and Eotaxin.
<5> The method according to any one of <1> to <4> above, further including measuring the amount of the cytokine in the sample derived from the evaluation subject.
<6> The method according to any one of <1> to <5> above, wherein the evaluating further includes evaluating whether the evaluation subject suffers from depression or whether the evaluation subject has possibility of development of depression by using as an indicator an amount of cortisol in the sample derived from the evaluation subject.
<7> The method according to <5> or <6> above, wherein the measuring further includes measuring the amount of the cortisol in the sample derived from the evaluation subject.
<8> The method according to <6> above, wherein the evaluating includes substituting data of the amounts of the cytokine and the cortisol into an evaluation formula that can evaluate, based on the data of the amounts of the cytokine and the cortisol, whether the evaluation subject suffers from depression or whether the evaluation subject has possibility of development of depression, and the evaluation formula includes at least two variables into which the data of the amounts of the cytokine and the cortisol are substituted.
<9> The method according to <3> above, wherein the evaluating includes substituting data of the amounts of two or more kinds of the cytokine into an evaluation formula that can evaluate, based on the data of the amounts of two or more kinds of the cytokine, whether the evaluation subject suffers from depression or whether the evaluation subject has possibility of development of depression, and the evaluation formula includes at least two variables into which the data of the amounts of two or more kinds of the cytokine are substituted.
<10> The method according to any one of <1> to <9> above, wherein the sample derived from the evaluation subject is saliva.
<11> A method for evaluating an effect of a therapy for depression in an evaluation subject, the method including
evaluating the effect of the therapy for depression by using as an indicator an amount of a cytokine in a sample derived from the evaluation subject.
<12> The method according to <11> above, wherein the evaluating includes a process to compare the amount of the cytokine in the sample derived from the evaluation subject, with a reference value,
   the reference value is: a value based on an amount of a cytokine in a sample derived from the evaluation subject and obtained before implementation of the therapy for depression; a value based on an amount of a cytokine in a sample derived from a patient with depression; or both of the values, and
   the evaluating is evaluating that the effect of the therapy for depression in the evaluation subject is low when:
      the amount of the cytokine in the sample derived from the evaluation subject is equal to or more than the value based on the amount of the cytokine in the sample derived from the evaluation subject and obtained before implementation of the therapy for depression;
      the amount of the cytokine in the sample derived from the evaluation subject is equal to or more than the value based on the amount of the cytokine in the sample derived from the patient with depression; or
      the amount of the cytokine in the sample derived from the evaluation subject is equal to or more than the value based on the amount of the cytokine in the sample derived from the evaluation subject and obtained before implementation of the therapy for depression and is equal to or more than the value based on the amount of the cytokine in the sample derived from the patient with depression.
<13> The method according to <11> above, wherein the evaluating includes substituting data of the amount of the cytokine into an evaluation formula that can evaluate the effect of the therapy for depression based on the data of the amount of the cytokine, and the evaluation formula includes at least one variable into which the data of the amount of the cytokine is substituted.
<14> The method according to any one of <11> to <13> above, wherein the cytokine is one or more kinds of cytokines selected from the group consisting of IL-1β, IL-7, IL-8, IL-9, IL-12 p70, MCP-1, IP-10, MIP-1α, TNF-α, IFN-γ, and Eotaxin.
<15> The method according to any one of <11> to <14> above, further including measuring the amount of the cytokine in the sample derived from the evaluation subject.
<16> The method according to any one of <11> to <15> above, wherein the evaluating further includes evaluating the effect of the therapy for depression by using as an indicator an amount of cortisol in the sample derived from the evaluation subject.
<17> The method according to <15> or <16> above, wherein the measuring further includes measuring the amount of the cortisol in the sample derived from the evaluation subject.
<18> The method according to <16> above, wherein the evaluating includes substituting data of the amounts of the cytokine and the cortisol into an evaluation formula that can evaluate the effect of the therapy for depression based on the data of the amounts of the cytokine and the cortisol, and the evaluation formula includes at least two variables into which the data of the amounts of the cytokine and the cortisol are substituted.
<19> The method according to <13> above, wherein the evaluating includes substituting data of the amounts of two or more kinds of the cytokine into an evaluation formula that can evaluate the effect of the therapy for depression based on the data of the amounts of two or more kinds of the cytokine, and the evaluation formula includes at least two variables into which the data of the amounts of two or more kinds of the cytokine are substituted.
<20> The method according to any one of <11> to <19> above, wherein the sample derived from the evaluation subject is saliva.
<21> A method for acquiring data for evaluating whether an evaluation subject suffers from depression or whether an evaluation subject has possibility of development of depression, or data for evaluating an effect of a therapy for depression in an evaluation subject, the method including
   measuring an amount of a cytokine in a sample derived from the evaluation subject.
<22> The method according to <21> above, wherein the cytokine is one or more kinds of cytokines selected from the group consisting of IL-1β, IL-7, IL-8, IL-9, IL-12 p70, MCP-1, IP-10, MIP-1α, TNF-α, IFN-γ, and Eotaxin.
<23> The method according to <21> or <22> above, further including measuring an amount of cortisol in the sample derived from the evaluation subject.
<24> The method according to any one of <21> to <23> above, wherein the sample derived from the evaluation subject is saliva.

## Claims

1. A method for evaluating whether an evaluation subject suffers from depression or whether an evaluation subject has possibility of development of depression, the method comprising
evaluating whether the evaluation subject suffers from depression or whether the evaluation subject has possibility of development of depression by using as an indicator an amount of a cytokine in a sample derived from the evaluation subject.

2. The method according to claim 1, wherein the evaluating comprises a process to compare the amount of the cytokine in the sample derived from the evaluation subject, with a standard value,
the standard value is: a value based on an amount of a cytokine in a sample derived from a healthy individual; a value based on an amount of a cytokine in a sample derived from a patient with depression; or both of the values, and
the evaluating is evaluating that the evaluation subject suffers from depression or has possibility of development of depression when:
the amount of the cytokine in the sample derived from the evaluation subject is more than the value based on the amount of the cytokine in the sample derived from the healthy individual;
the amount of the cytokine in the sample derived from the evaluation subject is equal to or more than the value based on the amount of the cytokine in the sample derived from the patient with depression; or
the amount of the cytokine in the sample derived from the evaluation subject is more than the value based on the amount of the cytokine in the sample derived from the healthy subject and is equal to or more than the value based on the amount of the cytokine in the sample derived from the patient with depression.

3. The method according to claim 1, wherein the evaluating comprises substituting data of the amount of the cytokine into an evaluation formula that can evaluate, based on the data of the amount of the cytokine, whether the evaluation subject suffers from depression or whether the evaluation subject has possibility of development of depression, and the evaluation formula includes at least one variable into which the data of the amount of the cytokine is substituted.

4. The method according to any one of claims 1 to 3, wherein the cytokine is one or more kinds of cytokines selected from the group consisting of IL-1β, IL-7, IL-8, IL-9, IL-12 p70, MCP-1, IP-10, MIP-1α, TNF-α, IFN-γ, and Eotaxin.

5. The method according to any one of claims 1 to 4, further comprising measuring the amount of the cytokine in the sample derived from the evaluation subject.

6. The method according to any one of claims 1 to 5, wherein the evaluating further comprises evaluating whether the evaluation subject suffers from depression or whether the evaluation subject has possibility of development of depression by using as an indicator an amount of cortisol in the sample derived from the evaluation subject.

7. The method according to claim 5 or 6, wherein the measuring further comprises measuring the amount of the cortisol in the sample derived from the evaluation subject.

8. The method according to any one of claims 1 to 7, wherein the sample derived from the evaluation subject is saliva.

9. A method for evaluating an effect of a therapy for depression in an evaluation subject, the method comprising
evaluating the effect of the therapy for depression by using as an indicator an amount of a cytokine in a sample derived from the evaluation subject.

10. The method according to claim 9, wherein the evaluating comprises a process to compare the amount of the cytokine in the sample derived from the evaluation subject, with a reference value,
the reference value is: a value based on an amount of a cytokine in a sample derived from the evaluation subject and obtained before implementation of the therapy for depression; a value based on an amount of a cytokine in a sample derived from a patient with depression; or both of the values, and
the evaluating is evaluating that the effect of the therapy for depression in the evaluation subject is low when:
the amount of the cytokine in the sample derived from the evaluation subject is equal to or more than the value based on the amount of the cytokine in the sample derived from the evaluation subject and obtained before implementation of the therapy for depression;
the amount of the cytokine in the sample derived from the evaluation subject is equal to or more than the value based on the amount of the cytokine in the sample derived from the patient with depression; or
the amount of the cytokine in the sample derived from the evaluation subject is equal to or more than the value based on the amount of the cytokine in the sample derived from the evaluation subject and obtained before implementation of the therapy for depression and is equal to or more than the value based on the amount of the cytokine in the sample derived from the patient with depression.

11. The method according to claim 9 or 10, wherein the cytokine is one or more kinds of cytokines selected from the group consisting of IL-1β, IL-7, IL-8, IL-9, IL-12 p70, MCP-1, IP-10, MIP-1α, TNF-α, IFN-γ, and Eotaxin.

12. The method according to any one of claims 9 to 11, wherein the evaluating further comprises evaluating the effect of the therapy for depression by using as an indicator an amount of cortisol in the sample derived from the evaluation subject.

13. The method according to any one of claims 9 to 12, wherein the sample derived from the evaluation subject is saliva.

14. A method for acquiring data for evaluating whether an evaluation subject suffers from depression or whether an evaluation subject has possibility of development of depression, or data for evaluating an effect of a therapy for depression in an evaluation subject, the method comprising
measuring an amount of a cytokine in a sample derived from the evaluation subject.

15. The method according to claim 14, wherein the cytokine is one or more kinds of cytokines selected from the group consisting of IL-1β, IL-7, IL-8, IL-9, IL-12 p70, MCP-1, IP-10, MIP-1α, TNF-α, IFN-γ, and Eotaxin.

16. The method according to claim 14 or 15, further comprising measuring an amount of cortisol in the sample derived from the evaluation subject.

17. The method according to any one of claims 14 to 16, wherein the sample derived from the evaluation subject is saliva.
